(19) Europäisches Patentamt / European Patent Office / Office européen des brevets

(11) **EP 4 362 812 B1**

(12) **EUROPEAN PATENT SPECIFICATION**

(45) Date of publication and mention
of the grant of the patent:
**09.07.2025 Bulletin 2025/28**

(21) Application number: **22761652.1**

(22) Date of filing: **30.06.2022**

(51) International Patent Classification (IPC):
***A61B 10/00*** *(2006.01)*

(52) Cooperative Patent Classification (CPC):
**A61B 10/0058; A61B 10/0096**

(86) International application number:
**PCT/US2022/035784**

(87) International publication number:
**WO 2023/278742 (05.01.2023 Gazette 2023/01)**

(54) **APPARATUS FOR COLLECTION OF FLUID SAMPLES**

VORRICHTUNG ZUR ENTNAHME VON FLÜSSIGKEITSPROBEN

APPAREIL POUR LA COLLECTE D'ÉCHANTILLONS DE FLUIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.06.2021 US 202163217025 P**

(43) Date of publication of application:
**08.05.2024 Bulletin 2024/19**

(73) Proprietor: **RSI Technology Group, LLC
Dallas, TX 75230 (US)**

(72) Inventor: **PENINGER, Diana
Flower Mound, TX 75022 (US)**

(74) Representative: **Abel & Imray LLP
Westpoint Building
James Street West
Bath BA1 2DA (GB)**

(56) References cited:
**EP-A1- 2 682 747      EP-A1- 3 521 202
EP-A1- 3 744 308      WO-A1-2018/222330**

# Description

## FIELD OF THE INVENTION

The present invention relates in general to the containment of biological fluids and, in specific embodiments, to collection of samples of biological fluids in relation to the field of assisted reproductive technologies. In particular, a method and apparatus for collection and preservation of fluid samples in accordance with the present invention results in collection of semen samples having improved viability both at the time of collection and after storage. The disclosed methods and apparatuses support a wide variety of applications for containment of or contact with biological fluids related to human and veterinary medicine and medical devices including, but not limited to, human reproductive medicine and animal husbandry.

## BACKGROUND OF THE INVENTION

Assisted reproductive technologies were developed originally to treat individuals with obstructed ovarian tubes, but have matured to procedures which, according to the U.S. Center for Disease Control (2013), now accounts for up to 2% of the annual U.S. birth rate. Since the first human birth from in vitro fertilization in 1978, there have been significant improvements in stimulation protocols, fertilization and culture techniques, use of donor gametes and embryos, and patient selection. Further, the use of pre-implantation genetic diagnosis/pre-implantation genetic screening, an invasive harvesting of cells for genetic screening, has allowed improved selection of embryos to avoid aneuploidy and other genetic defects. These improvements resulted in constantly increasing pregnancy rates while allowing a steady decrease in the number of embryos transferred (Center for Disease Control, 2013).

EP 3 744 308 A1 is considered the closest prior art and discloses a vessel for collecting semen comprising a collection means at the upper end of the vessel, a reservoir having an inner surface at the lower end of the vessel, an inner lid for engaging with the vessel and thereby sealing the reservoir, and an outer lid for sealable attachment to the vessel. U.S. Patent No. 6,864,046 discloses the use of a method for collecting the semen of an animal via a semen collection vessel having a semen extender solution capable of extending motility of collected semen. Such semen collection vessel is capable of extending the life of spermatozoa, and further improves conditions when utilized in connection with a semen extender solution, which may contain several ingredients including, but not limited to, nutrients to maintain its metabolic activity and to undergo the processes necessary for fertilization of the ova, proteins necessary for the sperm cells to grow and to mature into the spermatozoa, sugars to provide the sperm with energy, antimicrobial agents to reduce microbial contamination and prevent the spread of diseases that can be transported in the semen, and cryoprotectants to protect spermatozoa from damage due to ice crystal formation when frozen. This well-developed field has long-utilized semen extenders for improving the life of the collected samples. However, with the increase of cryopreservation, as well as the advancing state of the art in reproductive sciences, there remains a need for further enhancing the collection and storage of semen for improved semen quality and protection.

In addition to these traditional motivations, the recent pandemic has resulted in behavioral changes promoting a need for improved preservation of semen samples. It has been found that a shift for collection at clinic to at home for the population examined resulted in an increase in the time from collection to completed preparation. This has been shown to significantly effect the viability of useful sperm samples. Along with this there is the additional need for effective transportation or shipping of these collection and/or storage containers that provide for stability, containment, and security of the samples.

Therefore, there is a need for collection and/or storage containers that improve the viability of sperm, in collected semen and/or extend the time period for which such sperm remains viable.

## SUMMARY OF THE INVENTION

In general, the present disclosure relates to apparatuses and methods for collection, processing, and/or storage of biological fluids susceptible to degradation by oxidation. In particular, apparatuses and methods for collection and/or storage of mammalian semen samples are disclosed herein. The invention is further directed to apparatus, collections device and other medical devices that control free oxygen radicals within a biological fluid, and where this biological fluid is sperm, the amount of time available before a sperm must be used to fertilize an egg is increased.

In some embodiments, an apparatus for collection of fluids comprises a first vessel. The first vessel includes an upper portion and a lower portion. The upper portion is a wall, which can be of any suitable profile. The lower portion is a reservoir having a predetermined capacity. The wall and reservoir are joined such that the inner surface of the reservoir and the inner surface of the wall form a continuous surface. A suitable profile for the wall is one that will be effective as a collection means to cause liquid entering the top of the first vessel to collect in the reservoir by gravity flow.

In some embodiments, an apparatus for collection of fluids comprises a first vessel and a second vessel. In these embodiments, the first vessel is as described above, but additionally the first and second vessels are of sizes and shapes to permit the first vessel to be inserted into the second vessel. When so inserted, the upper edges of the first and second vessels are proximate to one another and are substantially congruent. In some embodiments, the upper edges of the first and second vessels are connected or secured to one other by a threaded connection, a snap connection, or other common means.

The apparatus comprises a reservoir seal means, wherein the reservoir has a first inner surface and the reservoir seal means has a bottom surface, such that when the reservoir seal means is installed, the first inner surface and the bottom surface circumscribe a sealed reservoir chamber

In some embodiments, in addition to any or all of the above attributes, the first vessel, second vessel, and lid can comprise a polymer, preferably a thermoplastic.

The foregoing has outlined rather broadly the features and technical advantages of the present invention in order that the detailed description of the invention that follows may be better understood. Additional features and advantages of the invention will be described hereinafter, which form the subject matter of the claims of the invention. It should be appreciated by those skilled in the art that the conception and specific embodiments disclosed may be readily utilized as a basis for modifying or designing other film structures and/or processes for carrying out the same purposes of the present invention. It should also be realized by those skilled in the art that such equivalent constructions may not depart from the scope of the invention as set forth in the appended claims. The novel features which are believed to be characteristic of the invention, both as to its structure and method of manufacture, together with further objects and advantages will be better understood from the following description.

BRIEF DESCRIPTION OF THE DRAWINGS

The present disclosure is illustrated by way of example, and not by way of limitation, in the accompanying drawings, in which like reference characters refer to like parts throughout, and in which:

FIGs. 1A and 1B shows vertical cross-section and expanded three-dimensional views of an embodiment of an apparatus comprising a single vessel;
FIGs. 2A and 2B shows vertical cross-section and expanded three-dimensional views of an embodiment of a reservoir seal in an apparatus comprising a single vessel;
FIGs. 3A and 3B shows vertical cross-section and expanded three-dimensional views of an embodiment of a reservoir seal in an apparatus comprising a single vessel;
FIGs. 4A and 4B shows vertical cross-section and expanded three-dimensional views of an embodiment of an apparatus comprising a first vessel and a second vessel;
FIGs. 5A and 5B shows vertical cross-section and expanded three-dimensional views of an embodiment of a reservoir seal in an apparatus comprising a first vessel and a second vessel; and
FIGs. 6A and 6B shows vertical cross-section and expanded three-dimensional views of an embodiment of a reservoir seal in an apparatus comprising a first vessel and a second vessel.

While the disclosed process and composition are susceptible to various modifications and alternative forms, the drawings illustrate specific embodiments herein described in detail by way of example. It should be understood, however, that the description herein of specific embodiments is not intended to limit the invention to the particular forms disclosed, but on the contrary, the intention is to cover all modifications, equivalents, and alternatives falling within the scope of the invention as defined by the appended claims.

DETAILED DESCRIPTION OF THE INVENTION

Illustrative embodiments of the subject matter claimed below will now be disclosed. In the interest of clarity, some features of some actual implementations may not be described in this specification. It will be appreciated that in the development of any such actual embodiments, numerous implementation-specific decisions must be made to achieve the developer's specific goals, such as compliance with system-related and business-related constraints, which will vary from one implementation to another. Moreover, it will be appreciated that such a development effort, even if complex and time-consuming, would be a routine undertaking for those of ordinary skill in the art having the benefit of this disclosure.

The words and phrases used herein should be understood and interpreted to have a meaning consistent with the understanding of those words and phrases by those skilled in the relevant art. No special definition of a term or phrase, i.e., a definition that is different from the ordinary and customary meaning as understood by those skilled in the art, is intended to be implied by consistent usage of the term or phrase herein. To the extent that a term or phrase is intended to have a special meaning, i.e., a meaning other than the broadest meaning understood by skilled artisans, such a special or clarifying definition will be expressly set forth in the specification in a definitional manner that provides the special or clarifying definition for the term or phrase. It must also be noted that, as used in the specification and the appended claims, the singular forms "a," "an," and "the" include plural references unless otherwise specified.

For example, the following discussion contains a non-exhaustive list of definitions of several specific terms used in this disclosure (other terms may be defined or clarified in a definitional manner elsewhere herein). These definitions are intended to clarify the meanings of the terms used herein. It is believed that the terms are used in a manner consistent with their ordinary meaning, but the definitions are nonetheless specified here for clarity.

Definitions

"Blow molding," as used herein, means a manufacturing process that allows hollow plastic parts to be formed. Air pressure is used to inflate soft plastic into a mold cavity. The three main types of blow molding are: extrusion blow molding, injection blow molding, and injection stretch blow molding.

"Injection molding," as used herein, means a manufacturing process where material is fed into a heated barrel where it is also melted. When smooth enough, the material is injected through a nozzle under pressure (filling cycle) to fill a mold cavity and then cools off (cooling cycle). Thereafter, the mold opens, and the part ejects.

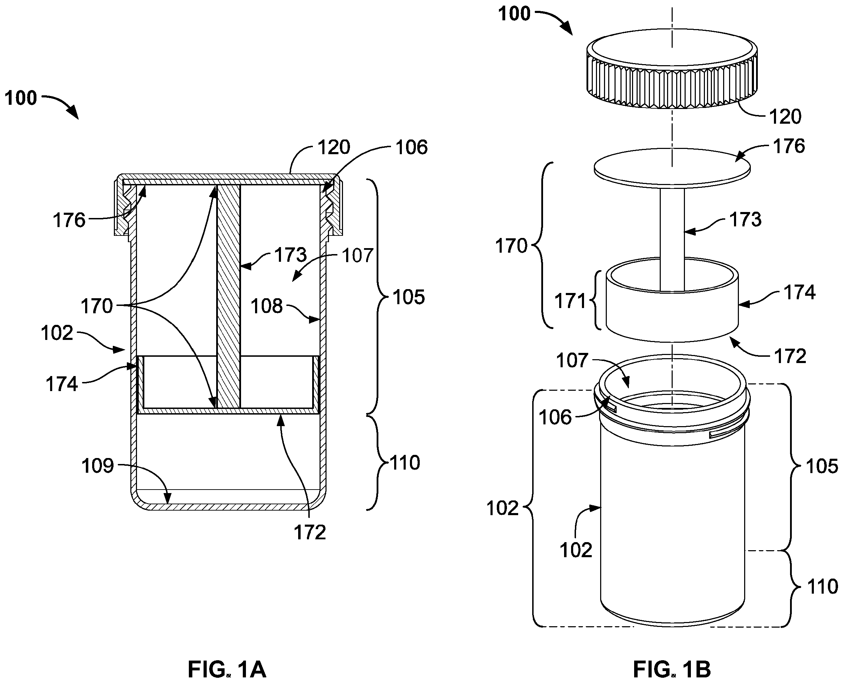

"Lower end," as used herein, with respect to the apparatus or component of the apparatus means the portion of apparatus or component of the apparatus, respectively, proximate to the bottom of the apparatus or component of the apparatus, respectively, when the apparatus is in the upright orientation resting on a horizontal surface. **FIGs. 1-6** each show embodiments of the apparatus in the upright orientation.

"Mate," as used herein, with respect to the apparatus means to form a connection between two components of the apparatus, such as between a lid and an edge or between two edges. Examples of such connections include, but are not limited to, overlapping edges, threaded connections, and tongue and groove connections. Connections can be self-securing, such as threaded connections or overlapping edges with interference such that the edges snap together. Alternatively, connections can be such that the components fit together but require external means such as tape to secure the connection.

"Thermoplastic," as used herein, means any polymer including but not limited to acrylonitrile butadiene styrene ("ABS"), polyamide ("PA"), polybutylene terephthalate ("PBT"), polycaprolactam, polycarbonate ("PC"), polyether ether ketone ("PEEK"), polyetherimide, polyethylene ("PE"; including ethylene homopolymers, such as, but not limited to, high density polyethylene and high-pressure, low density polyethylene; ethylene-alpha-olefin copolymers, such as, but not limited to, high density polyethylene, medium density polyethylene, and linear low density polyethylene; and copolymers of ethylene and polar comonomers, such as, but not limited to, ethylene-methyl-acrylate copolymer, ethylene-acrylic acid copolymer, ethylene-vinyl-acetate copolymer), polyethylene terephthalate ("PETP"), polymethyl methacrylate ("PMMA"), polyoxymethylene ("POM"), polyphenylene sulfide ("PPS"), polyphenylsulfone, polypropylene ("PP"), polystyrene ("PS"), polyvinylidene fluoride ("PVDF"), styrene acrylonitrile resin ("SAN"), thermoplastic elastomers ("TPE"), thermoplastic polyurethane ("TPU"), or combinations thereof.

"Upper end," as used herein, with respect to the apparatus or component of the apparatus means the portion of apparatus or component of the apparatus, respectively, proximate to the bottom of the apparatus or component of the apparatus, respectively, when the apparatus is in the upright orientation resting on a horizontal surface. **FIGs. 1-6** show embodiments of the apparatus in the upright orientation.

"Viability," as used herein, means a sperm sample that exceeds one or more lower reference limits for a semen sample of at least 1.5 ml: total sperm count greater than or equal to $39 \times 10^6$; sperm concentration greater than or equal to $15 \times 10^6$; total motility greater than or equal to 40%; progressive motility greater than or equal to 32%; vitality greater than or equal to 58%; and sperm morphology greater than or equal to 4%, (percent). "Selected viability," as used herein, means a one or more of the foregoing parameter selected by a user for determination of viability.

Physical Configuration of Apparatus

The apparatus comprises at least one vessel having a reservoir at the bottom of the vessel as the collection and storage of biological fluids, such as, but not limited to, mammalian semen. The apparatus further comprises a reservoir seal means, which encloses the reservoir after a fluid sample is collected. In some embodiments, the reservoir seal means comprises a seal element. In some embodiments, the reservoir seal means comprises a seal element connected to a positioning element. In some embodiments, the reservoir seal means comprises a seal element connected to a positioning element and the positioning element connected to a stabilizing element.

Without a reservoir seal, a first chamber is circumscribed by the inner surface of the reservoir, the inner surface of the collection means, and the inner surface of the lid. When the reservoir seal means is installed after collection of a fluid sample, a second chamber is formed, circumscribed by the inner surface of the reservoir and the bottom surface of the seal element. The second chamber has less volume than the first chamber and is circumscribed by less surface area than the first chamber.

When the apparatus is moved and/or transported, it is subjected to changes in external forces and/or changes in the orientation of the apparatus. Such changes in orientation can be one or more of tipping of the apparatus, inverting the apparatus, or even dropping the apparatus. Without a reservoir seal means, the fluid sample is subject to movement, sometimes severe movement, within the entire volume of the first chamber. With the reservoir seal means installed, movement of the fluid sample is restricted to the volume of the second chamber. Therefore, application of the same external forces on an apparatus with a reservoir seal means and an apparatus without a reservoir seal means would result in less trauma to and/or agitation of a fluid sample, since the reduced volume of the second chamber limits the distance that the sample can travel even when the apparatus is subjected to severe external forces. It is believed, without wishing to be bound by any particular theory, that less trauma to and/or agitation of a fluid sample, such as mammalian semen, helps to maintain the viability of such sample.

The reduced volume of the second chamber as compared to the first chamber also limits the amount of air to which a fluid sample is exposed during the time the sample is stored in the apparatus. It is believed, without wishing to be bound by any particular theory, that limiting the oxygen to which a sample of mammalian semen is exposed helps to maintain the viability of such sample. This becomes increasingly important as the time of storage increases.

The reduced surface area circumscribing the second chamber as compared to the first chamber also limits the amount of chemical reaction that can occur between a fluid sample and any compounds present on the surface area to which the fluid sample, such as mammalian semen, contacts. It is believed, without wishing to be bound by any particular theory, that limiting the surface area containing one or more compounds that are reactive with the fluid sample, such as mammalian, helps to maintain the viability of such sample.

In some embodiments, the material from which the reservoir, the collection means, lid, and/or seal element are fabricated comprise one or more antioxidants that counteract oxidative deterioration of the fluid sample, such as mammalian semen, due to exposure to oxygen. It is believed, without wishing to be bound by any particular theory, that the reduced size of the second chamber as compared to the first chamber promotes maintenance of a desired level of viability of a fluid sample such as mammalian semen by limiting reactants both in the vapor space and on the surface circumscribing the second chamber to which a fluid sample, such as mammalian semen, is exposed.

The reduced volume of the second chamber as compared to the first chamber also limits the change in temperature to which a fluid sample is exposed during the time the sample is stored in the apparatus. It is believed, without wishing to be bound by any particular theory, that restricting the movement of the fluid sample to a smaller volume provides more less variation in temperature of a fluid sample, such as mammalian semen, and helps to maintain the viability of such sample. This becomes increasingly important as the time of storage increases. This temperature stabilization effect is further enhanced when the first vessel is inserted into a second vessel as described elsewhere in this disclosure.

In some embodiments, the apparatus is a single vessel surrounded by one or more layers of insulation material. Such one or more layers can have uniform or variable thickness and can be: a) applied directly to the exterior surface of the first vessel, such as by coating or lamination; b) wrapped around the container; c) formed into an envelope, box, or other container suited for containment of the apparatus; or d) a combination thereof. In some embodiments, the apparatus comprises a first vessel and a second vessel, wherein the first vessel is configured to fit within the second vessel. The first vessel can be a close fit inside the second vessel or there can be an air gap between the outer surface of the first vessel and the inner surface of the second vessel. In some embodiments, the apparatus comprising either one vessel or two vessels, is inserted into a container fabricated from an insulating material having a thermal effusivity less than equal to that of a foamed polystyrene cup. In some embodiments, such container has a thermal effusivity of less than or equal to 1,000 $Ws^{0.5}/(m^2K)$, less than or equal to 500 $Ws^{0.5}/(m^2K)$, less than or equal to 250 $Ws^{0.5}/(m^2K)$, or in the range of from 100 $Ws^{0.5}/(m^2K)$ to 1 $Ws^{0.5}/(m^2K)$, where thermal effusivity "e" is calculated by the following equation:

$$e = \sqrt{(\lambda \rho c_p)}$$

wherein:

$\lambda$ is the thermal conductivity of the insulation material,
$\rho$ is the density of the insulation material, and
$c_p$ is the specific heat capacity of the insulation material.

In some embodiments, the first vessel is further placed inside a container fabricated from insulating material, as previously described, either or without an air gap and/or layer of insulation between the first vessel and the container. In addition to temperature control, such insulation, container, and/or packing material mitigate physical shock and trauma to a fluid sample stored in the apparatus caused by movement and/or mishandling of the containing holding the apparatus disclosed herein.

In some embodiments, a desired level of viability of a mammalian semen sample stored in an apparatus as described herein can be maintained for greater than or equal to 1 hour, greater than or equal to 6 hours, greater than or equal to 12 hours, greater than or equal to 24 hours, greater than or equal to 48 hours, or greater than or equal to 72 hours.

The ratio of the volume of the second chamber to the volume of the first chamber is less than or equal to 0.20, less than or equal to 0.10, less than or equal to 0.05, or less than or equal to 0.02.

When the reservoir sealing means is installed in the apparatus, the second chamber is circumscribed by a surface comprising the inner surface of the reservoir and the bottom surface of the seal element. The seal element has a sealing surface that engages with the first vessel proximate to the upper end of the reservoir and/or the lower end of the collection means. The inner surface of the reservoir and the inner surface of the collection means form a continuous inner surface of the first vessel. For purposes of this disclosure, the transition from the inner surface of the reservoir to the inner surface of the collection means is defined as the point at which the sealing surface of the seal element engages the continuous surface formed by the inner surface of the reservoir to the inner surface of the collection means.

The engagement between the sealing surface of the seal element and the inner surface of the first vessel can be implemented by positioning, friction, pressure, suction, or a combination thereof. In some embodiments positioning of the sealing surface of the seal element relative to the inner surface of the first vessel is maintained by a reservoir means comprising a seal element, a positioning element, and a stabilizing element. The configuration of the stabilizing element is such that the stabilizing element is secured by engagement of the lid with the first vessel, such as, but not limited to, by a threaded connection between the lid and the first vessel securing the perimeter of a disc-shaped stabilizing element between the upper edge of the first vessel and the lid. In some embodiments, the stabilizing element can be only a portion of a disc, wherein two or more contact points are provided for engagement between the upper edge of the first vessel and the lid. When the stabilizing element is secured between the upper edge of the first vessel and the lid, the sealing surface of the seal element is positioned close to the inner surface of the first vessel by connection of the seal element to the positioning element and connection of the positioning element to the stabilizing element. The fitment between the sealing surface of the seal element and the inner surface of the first vessel is such that the gap between the sealing surface of the seal element and the inner surface of the first vessel is less than the distance where the surface tension of a liquid sample, such as sample of mammalian semen, would overcome the forces of gravity is the apparatus was inverted such that the liquid sample would be retained in the second chamber upon such inversion.

In some embodiments, a frictional connection between the sealing surface of the seal element on the inner surface of the first vessel when a reservoir means, comprising a seal element and a positioning element, are engaged by manual application of force by a human or a machine through the positioning element connected to the seal element. Such force causes engagement between the sealing surface of the seal element and the inner surface of the first vessel by way of friction. Such engagement by friction is maintained by: a) taper of a frustoconical surface of the sealing surface of the seal element and/or taper of a frustoconical surface of the inner surface of the first vessel where the sealing surface of the seal element engages inner surface of the first vessel; b) texturing of the surface of a sealing surface of the seal element and/or a surface of the inner surface of the first vessel where the sealing surface of the seal element engages inner surface of the first vessel; c) pliability of the material from which the seal element and/or the first vessel are fabricated produce compressive forces in the seal element and/or the wall of the first vessel; or d) a combination thereof.

In some embodiments, pressure between the sealing surface of the seal element and the inner surface of the first vessel is maintained by a reservoir means comprising a seal element, a positioning element, and a stabilizing element. The configuration of the reservoir means is such that one or more of the seal element, the positioning element, and the stabilizing element is fabricated from a flexible material. In some embodiments, the stabilizing element is secured by engagement of the lid with the first vessel, such as, but not limited to, by a threaded connection between the lid and the first vessel securing the perimeter of a disc-shaped stabilizing element between the upper edge of the first vessel and the lid. In some embodiments, the stabilizing element can be only a portion of a disc, wherein two or more contact points are provided for engagement between the upper edge of the first vessel and the lid. When the stabilizing element is secured between the upper edge of the first vessel and the lid, the one or more of the seal element, the positioning element, and the stabilizing element are of a size and shape that one or more of the seal element, the positioning element, and the stabilizing element are cause to flex when the lid is fully engages with the upper edge of the first vessel. Such flexing of one or more of the seal element, the positioning element, and the stabilizing element produce a pressure between the sealing surface of the seal element and the inner surface of the first vessel.

In some embodiments, connection is maintained between the reservoir seal means and the first vessel by suction force. In such embodiments, the seal element is fabricated from a flexible material and configured such than when manual force by a human or a machine through the positioning element, the seal element is caused to flex and push a portion of the air in the second chamber to be removed by leakage between the interface of the sealing surface of the seal element and the inner surface of the first vessel. After such removal of a portion of the air from the second chamber, the air pressure outside the first chamber causes the seal element to remain flexed, thereby holding the reservoir means in place with an airtight connection at the interface of the sealing surface of the seal element and the inner surface of the first vessel.

In some embodiments, the seal between the sealing surface of the seal element and the inner surface of the first vessel is enhanced by fabrication of the seal element and/or the first vessel from plastic impregnated fabric and/or lamination of one or more layers plastic impregnated fabric to the seal element and/or inner surface of the first vessel, such as, but not limited to Celastic™ sheeting (available from Atlas International, California, USA).

In some embodiments, the configuration of the reservoir seal means is such that two or more of positioning, friction, pressure, and suction work in combination to maintain closure of the second chamber until the reservoir seal means is manually removed by a human or a machine from engagement with the inner surface of the first vessel by a human or a machine.

In some embodiments, the apparatus comprises a second vessel. The second vessel can be fabricated from the same or similar material as the first vessel or can be formed from a material providing a high level of insulation, and therefore temperature stabilization, such as but not limited to foamed polystyrene. In some embodiments, the apparatus comprises insulation material in a gap between the outer surface of the first vessel and the inner surface of the second vessel.

- Single-Vessel Embodiments

In some embodiments, as shown in **FIG. 1A** and **FIG. 1B,** the apparatus **100** comprises a single vessel. **FIG. 1A** and **FIG. 1B** show different views of the same apparatus **100** and accordingly use common reference numbers for components and portions of the apparatus **100. FIG. 1A** shows a vertical cross-section view of the apparatus **100** comprising vessel **102** and related components. **FIG. 1B** shows a corresponding three-dimensional expanded view of apparatus **100** comprising vessel **102** and its related components. The vessel **102** comprises at its upper end collection means **105** and at its lower end a reservoir **110**. The single vessel **102** in these embodiments is referred to elsewhere in this disclosure and in the claims as a first vessel.

The collection means **105** as shown if **FIG. 1A** and **FIG. 1B** is essentially a vertical wall, which is cylindrical in shape. However, the wall of the collection means **105,** when viewed as a vertical cross-section of the vessel can be straight, curved, sloped, or any combination thereof. When liquid is added to the vessel from above, the shape of the wall of the collection means **105** will either guide the flow of liquid by gravity or alternatively not restrict the flow by gravity of liquid toward the reservoir **110**.

When viewed as a horizontal cross-section, the wall of the collection means can be circular, oval-shaped, substantially square, substantially rectangular, irregularly shaped, or any other shape as can conveniently meet user preferences for ease of manufacturing, handling, and or storage of the apparatus. The horizontal cross-section can also be variable provided that the wall of the collection means guides fluids entering the top of the vessel by gravity flow toward the reservoir or does not restrict flow of fluids entering the top of the vessel toward the reservoir.

The collection means **105** has, at its upper end, an edge **106** which defines an opening **107**. Biological or bodily fluid is added to the apparatus through the opening **107** when the apparatus is used for collecting and storing the fluid, such as mammalian semen. The edge **106** and opening **107** shown in **FIG. 1A** and **FIG. 1B** are circular in shape. However, the edge **106** and opening **107** can be of any shape or size to accommodate convenient collection of semen samples from humans or from various mammalian animals.

In some embodiments, in addition to the above aspects of the physical configuration of the apparatus, the inner surface **108** of the wall of the collection means is configured to have an increased ratio of surface area of the inner surface **108** of the collection means to the volume of the collection means **105.** Means for increasing this ratio include, but are not limited to, one or more nubs, one or more circumferential ridges, one or more radial fins, or combinations thereof. Increasing the surface area increases the volume of fluid in direct contact with the surface on the apparatus.

The reservoir **110** has a volume suited for the amount of fluid to be collected in the apparatus. The volume for collection of semen is based on the amount of ejaculate from the species from which the semen will be collected, whether human or other mammalian animal.

In some embodiments, in addition to the above aspects of the physical configuration of the apparatus, the inner surface **109** of the reservoir is configured to have an increased ratio of surface area of the inner surface **109** of the reservoir to the volume of the reservoir **110.** Means for increasing this ratio include, but are not limited to, one or more nubs, one or more circumferential ridges, one or more radial fins, or combinations thereof.

In addition to the above aspects of the physical configuration of the apparatus, a closure means or lid **120** is provided to eliminate the possibility of spillage. The lid **120** shown in **FIG. 1A** and **FIG. 1B** is circular in shape. However, the lid **120** can be of any shape or size, provided that it is configured to mate with the edge **106** at the upper end of the collection means **105,** and to completely cover the opening **107**.

The apparatus **100** further includes a reservoir seal means **170**. The reservoir seal means **170** comprises: a) a seal element **171;** b) a seal element **171** and a positioning element **173;** or c) a seal element **171,** a positioning element **173,** and a stabilizing element **176.** The seal element **171** has at its lower end a bottom surface **172** surrounded by and adjacent to a sealing surface **174.** In some embodiments, comprises one or more positioning elements (not shown) attached to the bottom surface **172** of the seal element **171** that maintain a minimum distance between the bottom surface **172** of the seal element **171** and the bottom of the first vessel **102.**

In embodiments comprising a seal element **171,** a positioning element **173,** and a stabilizing element **176,** when stabilizing element **176** of the reservoir sealing means **170** is engaged between the upper edge **106** of the first vessel **102** and the lid **120,** the seal element **171** is positioned to form a second chamber circumscribed by the bottom surface of the seal element **171** and the inner surface of the reservoir **109.**

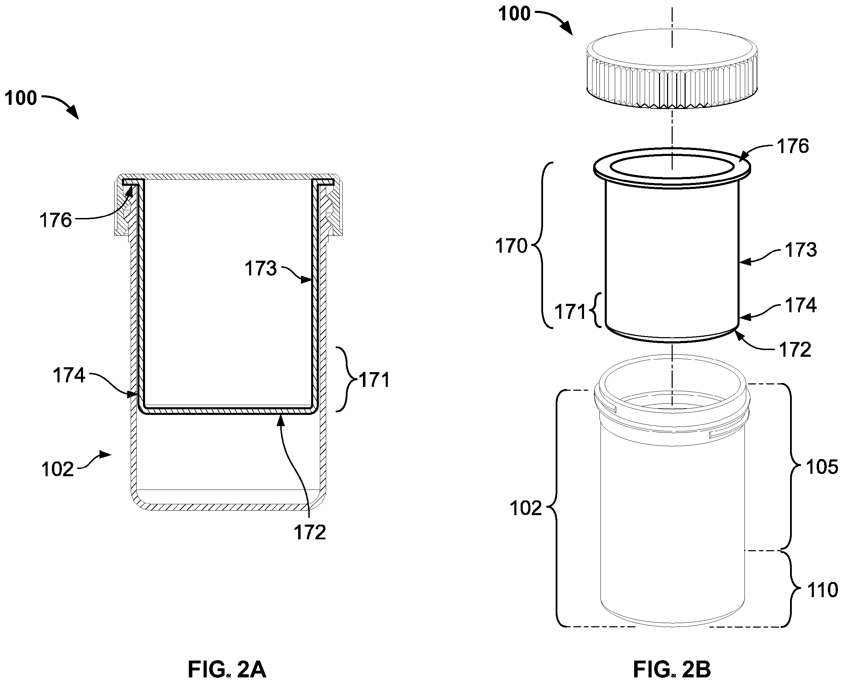

In some embodiments, as shown in **FIG. 2A** and **FIG. 2B,** the apparatus **100** comprises a single vessel similar to the embodiments shown in **FIG. 1A** and **FIG 1B. FIG. 2A** and **FIG. 2B** show different views of the same apparatus **100** and accordingly use common reference numbers for components and portions of the apparatus **100. FIG. 2A** shows a vertical cross-section view of the apparatus **100** comprising vessel **102** and related components. **FIG. 2B** shows a corresponding three-dimensional expanded view of apparatus **100** comprising vessel **102** and its related components. The vessel **102** comprises at its upper end collection means **105** and at its lower end a reservoir **110.** The single vessel **102** in these embodiments is referred to elsewhere in this disclosure and in the claims as a first vessel.

The apparatus **100** further includes a reservoir seal means **170** comprising a seal element **171,** a positioning element **173,** and a stabilizing element **176.** The seal element **171** has at its lower end a bottom surface **172** surrounded by and adjacent to a sealing surface **174.** In some embodiments, comprises one or more positioning elements (not shown) attached to the bottom surface **172** of the seal element **171** that maintain a minimum distance between the bottom surface **172** of the seal element **171** and the bottom of the first vessel **102.**

When stabilizing element **176** of the reservoir sealing means **170** is engaged between the upper edge **106** of the first vessel **102** and the lid **120,** the seal element **171** is positioned to form a second chamber circumscribed by the bottom surface of the seal element **171** and the inner surface of the reservoir **109.**

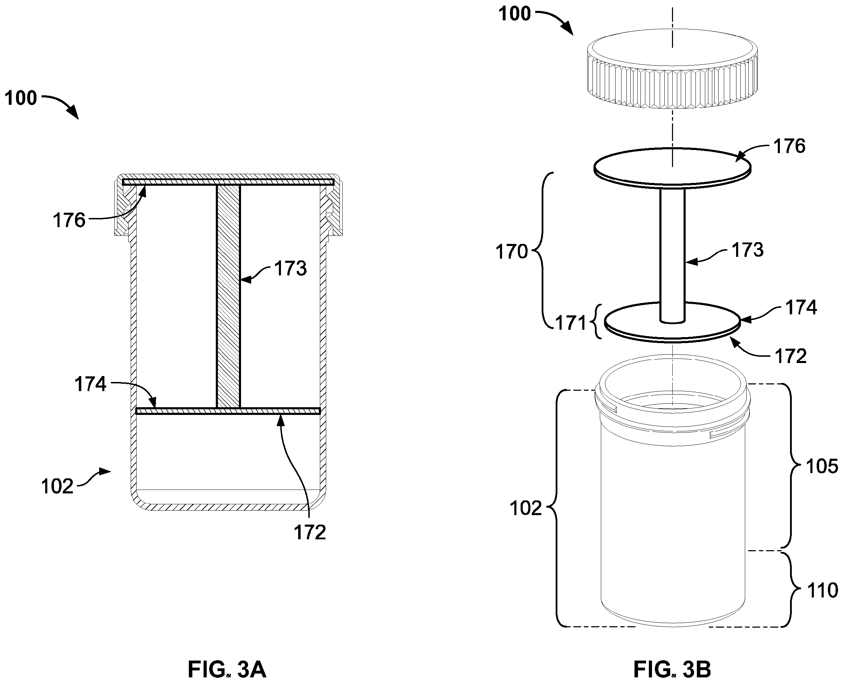

In some embodiments, as shown in **FIG. 3A** and **FIG. 3B,** the apparatus **100** comprises a single vessel similar to the embodiments shown in **FIG. 1A** and **FIG 1B. FIG. 3A** and **FIG. 3B** show different views of the same apparatus **100** and accordingly use common reference numbers for components and portions of the apparatus **100. FIG. 3A** shows a vertical cross-section view of the apparatus **100** comprising vessel **102** and related components. **FIG. 3B** shows a corresponding three-dimensional expanded view of apparatus **100** comprising vessel **102** and its related components. The vessel **102** comprises at its upper end collection means **105** and at its lower end a reservoir **110.** The single vessel **102** in these embodiments is referred to elsewhere in this disclosure and in the claims as a first vessel.

The apparatus **100** further includes a reservoir seal means **170** comprising a seal element **171,** a positioning element **173,** and a stabilizing element **176.** The seal element **171** has at its lower end a bottom surface **172** surrounded by and adjacent to a sealing surface **174.** In some embodiments, comprises one or more positioning elements (not shown) attached to the bottom surface **172** of the seal element **171** that maintain a minimum distance between the bottom surface **172** of the seal element **171** and the bottom of the first vessel **102.**

When stabilizing element **176** of the reservoir sealing means **170** is engaged between the upper edge **106** of the first vessel **102** and the lid **120,** the seal element **171** is positioned to form a second chamber circumscribed by the bottom surface of the seal element **171** and the inner surface of the reservoir **109.** In some embodiments, a portion of seal element **171** proximate to and sealing surface **174** comprises a pliable material to form an airtight seal between sealing surface **174** and the inner surface of the first vessel. In some embodiments, the first vessel is further placed inside a container fabricated from insulating material, as previously described, either or without an air gap and/or other packing material between the first vessel and the container.

- Two-Vessel Embodiments

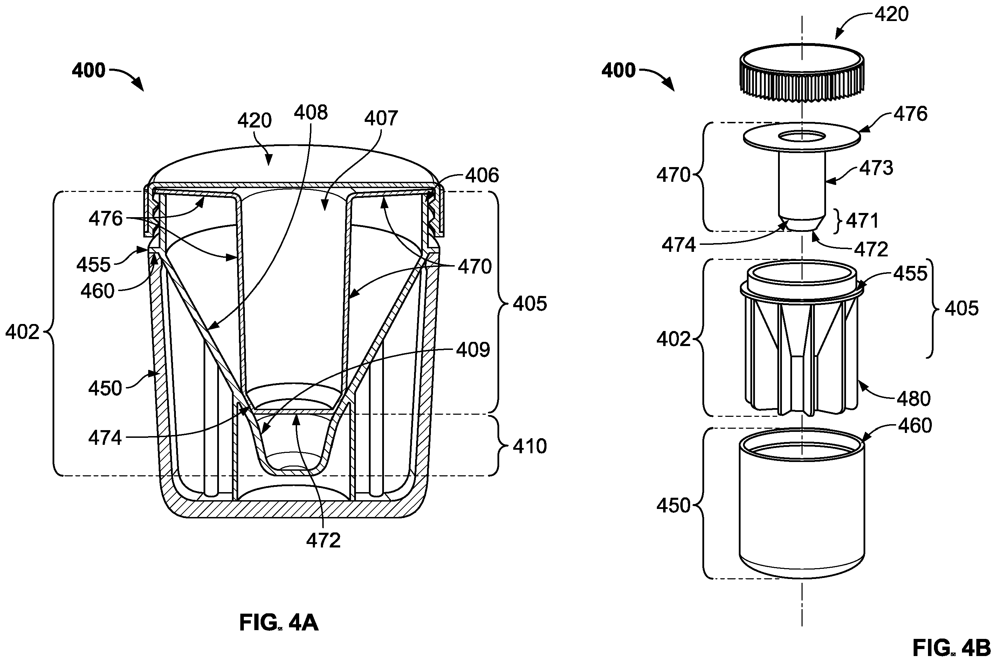

In some embodiments, as shown in **FIG. 4A** and **FIG. 4B,** the apparatus **400** comprises a first vessel **402** and a second vessel **450. FIG. 4A** and **FIG. 4B** show different views of the same apparatus **400** and accordingly use common reference numbers for components and portions of the apparatus **300. FIG. 4A** shows a vertical cross-section view of the apparatus **400** comprising vessel **402,** vessel **450,** and related components. **FIG. 4B** shows a corresponding three-dimensional expanded view of apparatus **400** comprising vessel **402,** vessel **450,** and related components. The vessel **402** comprises at its upper end collection means **405** and at its lower end a reservoir **410.** The vessel **402** in these embodiments is referred to elsewhere in this disclosure and in the claims as a first vessel.

Vessel **402** and vessel **450** are sized and shaped to allow vessel **402** to be inserted into vessel **450.** When so inserted, a support **455** proximate to the upper end of the collection means **405** is suited to mate with upper edge **460** of vessel **450.** The support **455** is suited to support vessel **402** substantially upright when vessel **402** is inserted into vessel **450** and when support **455** is resting on or mated with edge **460** of vessel **450.** In some embodiments, support **455** and edge **460** are connected or secured to one other by a threaded connection, a snap connection, or other convenient means. Additional support members **480,** including but not limited to fins as shown in **FIG. 4B,** can also be added to the outside of vessel **402** to provide additional support to maintain vessel **402** upright whether inserted in vessel **450** or placed on a flat surface. Support members can optionally be attached to vessel **450** or not attached to either vessel. In some embodiments, the inner surface of vessel **450** and the outer surface of vessel **402** form a chamber. In some embodiments, insulation means is installed in the chamber.

The vessel **450** in these embodiments is referred to elsewhere in this disclosure and in the claims as a second vessel.

The collection means **405** as shown if **FIG. 4A** and **FIG. 4B** is essentially a sloped wall, which is conical in shape. However, the wall of the collection means **405**, when viewed as a vertical cross-section of the vessel can be straight, curved, sloped, or any combination thereof. When liquid, a bodily fluid, is added to the vessel from above, the shape of the wall of the collection means **405** will either guide the flow of liquid by gravity or alternatively not restrict the flow by gravity of liquid toward the reservoir **410**.

When viewed as a horizontal cross-section, the wall of the collection means **405** can be circular, oval-shaped, substantially square, substantially rectangular, irregularly shaped, or any other shape as can conveniently meet user preferences for ease of manufacturing, handling, and or storage of the apparatus. The horizontal cross-section can also be variable provided that the wall of the collection means **405** guides fluids entering the top of the vessel by gravity flow toward the reservoir **410** or does not restrict flow of fluids entering the top of the vessel toward the reservoir **410**.

The collection means **405** has, at its upper end, an edge **406** which defines an opening **407**. Fluid is added to the apparatus through the opening **407** when the apparatus is used for collecting and storing fluid samples, such as mammalian semen. The edge **406** and opening **407** shown in **FIG. 4A** and **FIG. 4B** are circular in shape. However, the edge **406** and opening **407** can be of any shape or size to accommodate convenient collection of semen samples from humans or from various mammalian animals.

In some embodiments, in addition to the above aspects of the physical configuration of the apparatus, the inner surface **408** of the wall of the collection means is configured to have an increased ratio of surface area of the inner surface **408** of the collection means to the volume of the collection means **410**. Means for increasing this ratio include, but are not limited to, one or more nubs, one or more circumferential ridges, one or more radial fins, or combinations thereof.

The reservoir **410** has a volume suited for the amount of fluid to be collected in the apparatus. The volume for collection of semen is based on the amount of ejaculate from the species from which the semen sample will be collected, whether human or other mammalian animal.

In some embodiments, in addition to the above aspects of the physical configuration of the apparatus, the inner surface **409** of the reservoir is configured to have an increased ratio of surface area of the inner surface **409** of the reservoir to the volume of the reservoir **410**. Means for increasing this ratio include, but are not limited to, one or more nubs, one or more circumferential ridges, one or more radial fins, or combinations thereof.

In addition to the above aspects of the physical configuration of the apparatus, a closure means or lid **420** is provided. The lid **420** shown in **FIG. 4A** and **FIG. 4B** is circular in shape. However, the lid **420** can be of any shape or size, provided that it is configured to mate with the edge **406** at the upper end of the collection means **405** and to completely cover the opening **407**.

The apparatus further includes a reservoir seal means **470**. The reservoir seal means **470** comprises: a) a seal element **471**; b) a seal element **471** and a positioning element **473**; or c) a seal element **471**, a positioning element **473**, and a stabilizing element **476**. The seal element **471** has at its lower end a bottom surface **472** surrounded by and adjacent to a sealing surface **471**. In some embodiments, comprises one or more positioning elements (not shown) attached to the bottom surface **472** of the seal element **471** that maintain a minimum distance between the bottom surface **172** of the seal element **471** and the bottom of the first vessel **402**.

In embodiments comprising a seal element **471**, a positioning element **473**, and a stabilizing element **476**, when stabilizing element **476** of the reservoir sealing means **470** is engaged between the upper edge **406** of the first vessel **402** and the lid **420**, the seal element **471** is positioned to form a second chamber circumscribed by the bottom surface of the seal element **174** and the inner surface of the reservoir **409**.

In some embodiments, stabilizing element **476** is flexible and causes the sealing surface **474** to engage the first vessel proximate the junction between the reservoir **410** and the collection means **405** such that the inner surface **409** of the reservoir and the bottom surface **472** of the seal element define an enclosed chamber. When the lid **420** is fully installed, stabilizing element **476** flexes to provide force to create and maintain a seal between the sealing surface **474** and the first vessel **402** and thereby retain a fluid sample in the enclosed chamber defined by the bottom surface **472** of the reservoir seal means **470** and the inner surface the first vessel **402** regardless of the spatial orientation of the apparatus **400**. The reservoir seal means **470** shown in **FIG. 4A** and **FIG. 4B** has a cylindrical body with a bottom surface **472** surrounded by a sealing surface **474** at its lower end and a stabilizing element **476** comprising a flexible flange at its upper end as a means for providing sealing force. Full closure of the lid **420** causes the flange to flex to provide the seal between the sealing surface **474** and the inner surface of the first vessel **402**. Sealing force can also be provided without the flexible flange by selecting a length for the positioning element **473** of that causes an interference fit of reservoir seal means **476** between the lid **420**, when fully engaged, and the inner surface of the first vessel **402**, such sealing force being produced by flexure of the lid **420** and/or compression of one or more of the seal element **471**, positioning element **373**, and stabilizing element **376**. The positioning element **373** is not restricted to the two configurations described above and can be any configuration such as, but not limited to, one or more columns, a slotted hollow cylinder, a cylinder with vertical corrugations, a cylinder with horizontal corrugations, a flexible bellows, a cylindrical member of variable horizontal cross-section, or any other configuration provided that a seal between the sealing surface **374** of the seal element **471** and the inner surface of first vessel **402** is induced by engagement of the lid **420** to the inner surface of first vessel **402**. In other embodiments, the reservoir seal means can be independent of interaction with the lid, such as, but not limited to a plug or stopper engaging the sealing surface **474** by interference fit.

In other embodiments, reservoir seal means **470** comprises a seal element **471**, optionally connected to a positioning element **473**, without a stabilizing element **476**. In such embodiments, the reservoir seal means can be independent of interaction with the lid **420**, such as, but not limited to a seal element **471** comprising a plug or stopper engaging the sealing surface **474** and the inner surface of the first vessel by friction and/or suction, as described above.

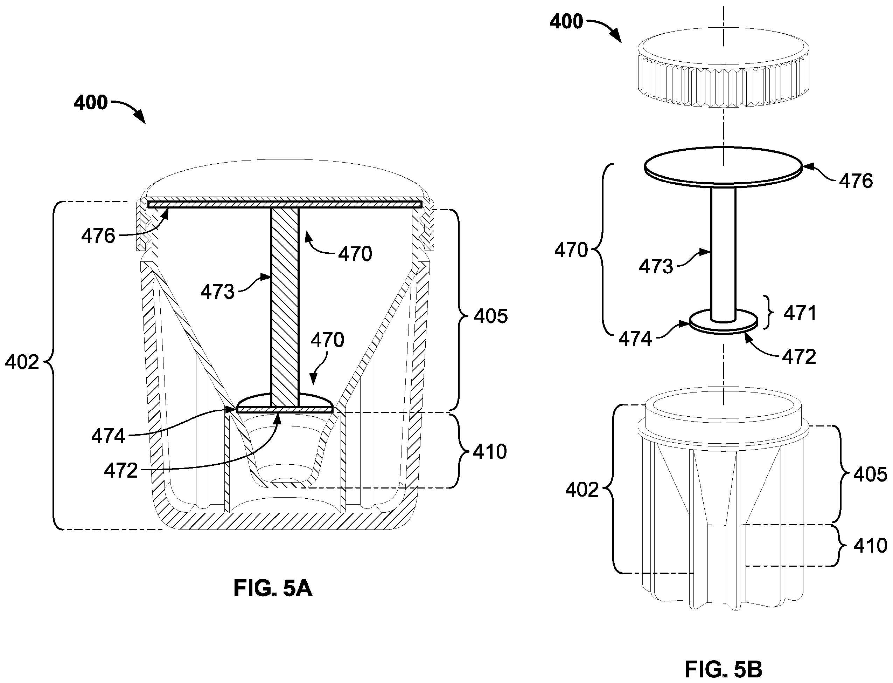

In some embodiments, as shown in **FIG. 5A** and **FIG. 5B**, the apparatus **400** comprises a single vessel similar to the embodiments shown in **FIG. 1A** and **FIG 1B**. **FIG. 5A** and **FIG. 5B** show different views of the same apparatus **400** and accordingly use common reference numbers for components and portions of the apparatus **400**. **FIG. 5A** shows a vertical cross-section view of the apparatus **400** comprising vessel **402** and related components. **FIG. 5B** shows a corresponding three-dimensional expanded view of apparatus **400** comprising vessel **402** and its related components. The vessel **402** comprises at its upper end collection means **405** and at its lower end a reservoir **410**. The single vessel **402** in these embodiments is referred to elsewhere in this disclosure and in the claims as a first vessel.

The apparatus **400** further includes a reservoir seal means **470** comprising a seal element **471**, a positioning element **473**, and a stabilizing element **476**. The seal element **471** has at its lower end a bottom surface **472** surrounded by and adjacent to a sealing surface **474**. In some embodiments, comprises one or more positioning elements (not shown) attached to the bottom surface **472** of the seal element **471** that maintain a minimum distance between the bottom surface **472** of the seal element **471** and the bottom of the first vessel **402**.

When stabilizing element **476** of the reservoir sealing means **470** is engaged between the upper edge **406** of the first vessel **402** and the lid **420**, the seal element **471** is positioned to form a second chamber circumscribed by the bottom surface of the seal element **471** and the inner surface of the reservoir **409**.

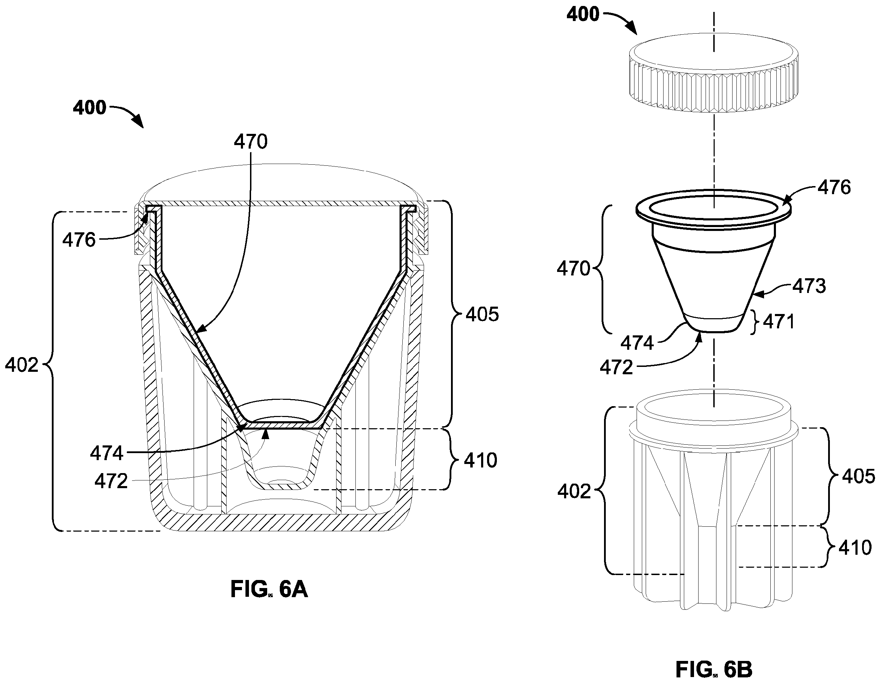

In some embodiments, as shown in **FIG. 6A** and **FIG. 6B**, the apparatus **400** comprises a single vessel similar to the embodiments shown in **FIG. 1A** and **FIG 1B**. **FIG. 6A** and **FIG. 6B** show different views of the same apparatus **400** and accordingly use common reference numbers for components and portions of the apparatus **400**. **FIG. 6A** shows a vertical cross-section view of the apparatus **400** comprising vessel **402** and related components. **FIG. 6B** shows a corresponding three-dimensional expanded view of apparatus **400** comprising vessel **402** and its related components. The vessel **402** comprises at its upper end collection means **405** and at its lower end a reservoir **410**. The single vessel **402** in these embodiments is referred to elsewhere in this disclosure and in the claims as a first vessel.

The apparatus **400** further includes a reservoir seal means **470** comprising a seal element **471**, a positioning element **473**, and a stabilizing element **476**. The seal element **471** has at its lower end a bottom surface **472** surrounded by and adjacent to a sealing surface **474**. In some embodiments, comprises one or more positioning elements (not shown) attached to the bottom surface **472** of the seal element **471** that maintain a minimum distance between the bottom surface **472** of the seal element **471** and the bottom of the first vessel **402**.

When stabilizing element **476** of the reservoir sealing means **470** is engaged between the upper edge **406** of the first vessel **402** and the lid **420**, the seal element **471** is positioned to form a second chamber circumscribed by the bottom surface of the seal element **471** and the inner surface of the reservoir **409**. In some embodiments, a portion of seal element **471** proximate to and sealing surface **474** comprises a pliable material to form an airtight seal between sealing surface **474** and the inner surface of the first vessel.

In some embodiments, the apparatus **400** further comprises insulation materials wherein the insulation material provides more protection from heat entering or leaving a fluid sample stored in the reservoir **410**.

Various embodiments of the invention include, but are not limited to:

> In some embodiments, the apparatus further comprises a second vessel, wherein the first vessel is inserted into the second vessel.
>
> In some embodiments the first inner surface comprises geometric surface features suited to increase the ratio of the area if the first inner surface to the predetermined capacity.
>
> In some embodiments, the first vessel, the second vessel, the reservoir seal means, and the lid are each independently comprised of acrylonitrile butadiene styrene, polyamide, polybutylene terephthalate, polycaprolactam, polycarbonate, polyether ether ketone, polyetherimide, polyethylene (including ethylene homopolymers, such as, but not limited to, high density polyethylene and high-pressure, low density polyethylene; ethylene-alpha-olefin copolymers, such as, but not limited to, high density polyethylene, medium density polyethylene, and linear low density polyethylene; and copolymers of ethylene and polar comonomers, such as, but not limited to, ethylene-methyl-acrylate copolymer, ethylene-acrylic acid copolymer, ethylene-vinyl-acetate copolymer), polyethylene terephthalate, polymethyl methacrylate, polyoxymethylene, polyphenylene sulfide, polyphenylsulfone, polypropylene, polystyrene, polyvinylidene fluoride, styrene acrylonitrile resin, thermoplastic elastomers, thermoplastic polyurethane, or combinations thereof.

In some embodiments, the apparatus comprises a first vessel having a wall attached at its lower end to a reservoir and having a first edge at its upper end; and a support means suitable for maintaining the first vessel in an upright orientation; wherein the reservoir and the wall form a first inner surface circumscribing a chamber within the first vessel.

For the sake of brevity, only certain ranges are explicitly disclosed herein. However, in addition to recited ranges, any lower limit may be combined with any upper limit to recite a range not explicitly recited, as well as, ranges from any lower limit may be combined with any other lower limit to recite a range not explicitly recited, in the same way, ranges from any upper limit may be combined with any other upper limit to recite a range not explicitly recited. Additionally, within a range includes every point or individual value between its end points even though not explicitly recited. Thus, every point or individual value may serve as its own lower or upper limit combined with any other point or individual value or any other lower or upper limit, to recite a range not explicitly recited.

Although the present invention and its advantages have been described in detail, it should be understood that various changes, substitutions and alterations can be made herein without departing from the scope of the invention as defined by the appended claims. Moreover, the scope of the present application is not intended to be limited to the particular embodiments of the apparatuses, methods, compositions, and/or devices described in the specification. As one of the ordinary skill in the art will readily appreciate from the disclosure of the present invention, apparatuses, methods, compositions, and/or devices, presently existing or later to be developed that perform substantially the same function or achieve substantially the same result as the corresponding embodiments described herein, may be utilized according to the present invention.

**Claims**

1. A vessel (102) for collection and preservation of fluid samples, comprising:

   a. a collection means (105) at the upper end of the vessel;
   b. a reservoir (110) at the lower end of the vessel (102),
   c. a reservoir seal means (170); and
   d. a lid (120) suited for sealable attachment to the vessel (102):

   wherein:

   the reservoir (110) comprises an inner surface (109);

   the reservoir seal means (170) is configured to engage with the vessel (102) to enclose the reservoir (110); and **characterised in that**:
   the inner surface (108) of the vessel (102) and the inner surface of the lid (120) circumscribe a first chamber;
   the inner surface (109) of the reservoir (110) and a bottom surface of the reservoir seal means (170) circumscribe a second chamber; and
   the ratio of the volume of the second chamber to the volume of the first chamber is less than or equal to 0.20.

2. The vessel (102) of claim 1, wherein the reservoir seal means (170) comprises a seal element (171).

3. The vessel (102) of claim 2, wherein the reservoir (102), the collection means (105), the lid (120), and/or the seal element (171) are fabricated from material comprising one or more antioxidants that counteract oxidative deterioration of a mammalian semen sample.

4. The vessel (102) of claim 2 or 3, wherein:

   a. the seal element (171) comprises a sealing surface (174) ;
   b. the vessel (102) has an inner surface (108); and
   c. the sealing surface (174) of the seal element (171) engages with the inner surface (108) of thefrrst vessel (102) by positioning, friction, pressure, suction, or a combination thereof.

5. The vessel (102) of claim 4, wherein engagement of the sealing surface (174) of the seal element (171) with the inner surface (108) of the vessel (102) forms an airtight connection.

6. The vessel (102) of claim 4 or 5, wherein the reservoir seal means (170) further comprises a positioning element (173).

7. The vessel (102) of claim 6, wherein the reservoir seal means (170) further comprises a stabilizing element (176).

8. The vessel (102) of any one of the preceding claims, further comprising an external layer of insulation.

9. An apparatus (400) for collection and preservation of fluid samples, comprising:

   a. a first vessel (402) having a collection means (405) at the upper end of the first vessel (402) and a reservoir (410) at the lower end of the first vessel (402), and

b. a second vessel (450), wherein the first vessel (402) is inserted into the second vessel (450);
c. a lid (420) suited for sealable attachment to the first vessel (402); and
d. a reservoir seal means (470);

wherein:

the reservoir (410) comprises an inner surface (102);
the reservoir seal means (470) is configured to engage with the first vessel (402) to enclose the reservoir (410); and **characterised in that**:
the inner surface (408) of the first vessel (402) and the inner surface of the lid (420) circumscribe a first chamber;
the inner surface (409) of the reservoir (410) and a bottom surface of the reservoir seal means (470) circumscribe a second chamber; and
the ratio of the volume of the second chamber to the volume of the first chamber is less than or equal to 0.20.

10. The apparatus (400) of claim 9, wherein the reservoir seal means (470) comprises a seal element (471).

11. The apparatus (400) of claim 9 or 10, wherein the reservoir (402), the collection means (405), the lid (420), and/or the seal element (471) are fabricated from material comprising one or more antioxidants that counteract oxidative deterioration of a mammalian semen sample.

12. The apparatus (400) of claim 11, wherein:

a. the seal element (471) comprises a sealing surface (474) ;
b. the first vessel (402) has an inner surface (408); and
c. the sealing surface (474) of the seal element (471) engages with the inner surface (408) of the first vessel (402) by positioning, friction, pressure, suction, or a combination thereof.

13. The apparatus (400) of claim 12, wherein engagement of the sealing surface (474) of the seal element (471) with the inner surface (408) of the first vessel (402) forms an airtight connection.

14. The apparatus (400) of claim 13, wherein the reservoir seal means (470) further comprises a positioning element (473).

15. The apparatus (400) of claim 14, wherein the reservoir seal means (470) further comprises a stabilizing element (476).

16. The apparatus (400) of any one of claims 9 through 15, further comprising a layer of insulation, an air gap, or a combination thereof between the first vessel (402) and the second vessel (450).

17. The apparatus (400) of any one of claims 9 through 16, further comprising a layer of insulation surrounding the apparatus.

**Patentansprüche**

1. Behälter (102) zur Sammlung und Konservierung von Flüssigkeitsproben, aufweisend:

a. ein Sammelmittel (105) am oberen Ende des Behälters,
b. ein Reservoir (110) am unteren Ende des Behälters (102),
c. ein Reservoir-Dichtungselement (170), und
d. einen Deckel (120), der zur abdichtbaren Befestigung an dem Behälter (102) geeignet ist, wobei:

das Reservoir (110) eine innere Oberfläche (109) aufweist,
das Reservoir-Dichtungselement (170) so ausgebildet ist, dass es mit dem Behälter (102) in Eingriff steht, um das Reservoir (110) zu verschließen, und **dadurch gekennzeichnet ist, dass**:

die innere Oberfläche (108) des Behälters (102) und die innere Oberfläche des Deckels (120) eine erste Kammer umschließen,
die innere Oberfläche (109) des Reservoirs (110) und eine Bodenfläche des Reservoir-Dichtungselements (170) eine zweite Kammer umschließen, und
das Verhältnis des Volumens der zweiten Kammer zum Volumen der ersten Kammer kleiner oder gleich 0,20 ist.

2. Behälter (102) nach Anspruch 1, wobei das Reservoir-Dichtungselement (170) ein Dichtungselement (171) aufweist.

3. Behälter (102) nach Anspruch 2, wobei das Reservoir (102), das Sammelmittel (105), der Deckel (120) und/oder das Dichtungselement (171) aus Material gefertigt sind, das ein oder mehrere Antioxidantien aufweist, die den oxidativen Abbau einer Säugetiersamenprobe verhindern.

4. Behälter (102) nach Anspruch 2 oder 3, wobei:

a. das Dichtungselement (171) eine Dichtfläche (174) aufweist,

b. der Behälter (102) eine innere Oberfläche (108) aufweist, und

c. die Dichtfläche (174) des Dichtungselements (171) mit der inneren Oberfläche (108) des Behälters (102) durch Positionierung, Reibung, Druck, Sog oder eine Kombination davon in Eingriff steht.

5. Behälter (102) nach Anspruch 4, wobei der Eingriff der Dichtfläche (174) des Dichtungselements (171) mit der inneren Oberfläche (108) des Behälters (102) eine luftdichte Verbindung bildet.

6. Behälter (102) nach Anspruch 4 oder 5, wobei das Reservoir-Dichtungselement (170) weiterhin ein Positionierungselement (173) aufweist.

7. Behälter (102) nach Anspruch 6, wobei das Reservoir-Dichtungselement (170) weiterhin ein Stabilisierungselement (176) aufweist.

8. Behälter (102) nach einem der vorhergehenden Ansprüche, der weiterhin eine äußere Isolierschicht aufweist.

9. Vorrichtung (400) zur Sammlung und Konservierung von Flüssigkeitsproben, aufweisend:

    a. einen ersten Behälter (402) mit einem Sammelmittel (405) am oberen Ende des ersten Behälters (402) und einem Reservoir (410) am unteren Ende des ersten Behälters (402), und

    b. einen zweiten Behälter (450), wobei der erste Behälter (402) in den zweiten Behälter (450) eingesetzt ist,

    c. einen Deckel (420), der zur abdichtbaren Befestigung an dem ersten Behälter (402) geeignet ist, und

    d. ein Reservoir-Dichtungselement (470), wobei:

        das Reservoir (410) eine innere Oberfläche (102) aufweist,

        das Reservoir-Dichtungselement (470) so ausgebildet ist, dass es mit dem ersten Behälter (402) in Eingriff steht, um das Reservoir (410) zu verschließen, und **dadurch gekennzeichnet ist, dass**:

            die innere Oberfläche (408) des ersten Behälters (402) und die innere Oberfläche des Deckels (420) eine erste Kammer umschließen,

            die innere Oberfläche (409) des Reservoirs (410) und eine Bodenfläche des Reservoir-Dichtungselements (470) eine zweite Kammer umschließen, und

das Verhältnis des Volumens der zweiten Kammer zum Volumen der ersten Kammer kleiner oder gleich 0,20 ist.

10. Vorrichtung (400) nach Anspruch 9, wobei das Reservoir-Dichtungselement (470) ein Dichtungselement (471) aufweist.

11. Vorrichtung (400) nach Anspruch 9 oder 10, wobei das Reservoir (402), das Sammelmittel (405), der Deckel (420) und/oder das Dichtungselement (471) aus Material gefertigt sind, das ein oder mehrere Antioxidantien aufweist, die den oxidativen Abbau einer Säugetiersamenprobe verhindern.

12. Vorrichtung (400) nach Anspruch 11, wobei:

    a. das Dichtungselement (471) eine Dichtfläche (474) aufweist,

    b. der erste Behälter (402) eine innere Oberfläche (408) aufweist, und

    c. die Dichtfläche (474) des Dichtungselements (471) mit der inneren Oberfläche (408) des ersten Behälters (402) durch Positionierung, Reibung, Druck, Sog oder eine Kombination davon in Eingriff steht.

13. Vorrichtung (400) nach Anspruch 12, wobei der Eingriff der Dichtfläche (474) des Dichtungselements (471) mit der inneren Oberfläche (408) des ersten Behälters (402) eine luftdichte Verbindung bildet.

14. Vorrichtung (400) nach Anspruch 13, wobei das Reservoir-Dichtungselement (470) weiterhin ein Positionierungselement (473) aufweist.

15. Vorrichtung (400) nach Anspruch 14, wobei das Reservoir-Dichtungselement (470) weiterhin ein Stabilisierungselement (476) aufweist.

16. Vorrichtung (400) nach einem der Ansprüche 9 bis 15, die weiterhin eine Isolierschicht, einen Luftspalt oder eine Kombination davon zwischen dem ersten Behälter (402) und dem zweiten Behälter (450) aufweist.

17. Vorrichtung (400) nach einem der Ansprüche 9 bis 16, die weiterhin eine Isolierschicht um die Vorrichtung herum aufweist.

**Revendications**

1. Récipient (102) pour la collecte et la conservation d'échantillons de fluide, comprenant :

    a. des moyens de collecte (105) au niveau de l'extrémité supérieure du récipient ;

b. un réservoir (110) au niveau de l'extrémité inférieure du récipient (102),

c. des moyens d'étanchéité de réservoir (170) ; et

d. un couvercle (120) adapté pour une fixation pouvant être scellée au récipient (102) : dans lequel :

le réservoir (110) comprend une surface intérieure (109) ;
les moyens d'étanchéité de réservoir (170) sont configurés pour venir en prise avec le récipient (102) pour enfermer le réservoir (110) ; et **caractérisé en ce que** :

la surface intérieure (108) du récipient (102) et la surface intérieure du couvercle (120) entourent une première chambre ;
la surface intérieure (109) du réservoir (110) et une surface inférieure des moyens d'étanchéité de réservoir (170) entourent une seconde chambre ; et
le rapport du volume de la seconde chambre au volume de la première chambre est inférieur ou égal à 0,20.

2. Récipient (102) selon la revendication 1, dans lequel les moyens d'étanchéité de réservoir (170) comprennent un élément d'étanchéité (171).

3. Récipient (102) selon la revendication 2, dans lequel le réservoir (102), les moyens de collecte (105), le couvercle (120) et/ou l'élément d'étanchéité (171) sont fabriqués à partir d'un matériau comprenant un ou plusieurs antioxydants qui agissent à l'encontre d'une détérioration oxydative d'un échantillon de sperme de mammifère.

4. Récipient (102) selon la revendication 2 ou 3, dans lequel :

a. l'élément d'étanchéité (171) comprend une surface d'étanchéité (174) ;
b. le récipient (102) présente une surface intérieure (108) ; et
c. la surface d'étanchéité (174) de l'élément d'étanchéité (171) vient en prise avec la surface intérieure (108) du récipient (102) par positionnement, frottement, pression, aspiration, ou une combinaison de ceux-ci.

5. Récipient (102) selon la revendication 4, dans lequel la mise en prise de la surface d'étanchéité (174) de l'élément d'étanchéité (171) avec la surface intérieure (108) du récipient (102) forme une connexion étanche à l'air.

6. Récipient (102) selon la revendication 4 ou 5, dans lequel les moyens d'étanchéité de réservoir (170) comprennent en outre un élément de positionnement (173).

7. Récipient (102) selon la revendication 6, dans lequel les moyens d'étanchéité de réservoir (170) comprennent en outre un élément de stabilisation (176).

8. Récipient (102) selon l'une quelconque des revendications précédentes, comprenant en outre une couche d'isolation externe.

9. Appareil (400) pour la collecte et la conservation d'échantillons de fluide, comprenant :

a. un premier récipient (402) présentant des moyens de collecte (405) au niveau de l'extrémité supérieure du premier récipient (402) et un réservoir (410) au niveau de l'extrémité inférieure du premier récipient (402), et
b. un second récipient (450), dans lequel le premier récipient (402) est inséré dans le second récipient (450) ;
c. un couvercle (420) adapté pour une fixation pouvant être scellée au premier récipient (402) ; et
d. des moyens d'étanchéité de réservoir (470) ;

dans lequel :

le réservoir (410) comprend une surface intérieure (102) ;
les moyens d'étanchéité de réservoir (470) sont configurés pour venir en prise avec le premier récipient (402) pour enfermer le réservoir (410) ; et **caractérisé en ce que** :

la surface intérieure (408) du premier récipient (402) et la surface intérieure du couvercle (420) entourent une première chambre ;
la surface intérieure (409) du réservoir (410) et une surface inférieure des moyens d'étanchéité de réservoir (470) entourent une seconde chambre ; et
le rapport du volume de la seconde chambre au volume de la première chambre est inférieur ou égal à 0,20.

10. Appareil (400) selon la revendication 9, dans lequel les moyens d'étanchéité de réservoir (470) comprennent un élément d'étanchéité (471).

11. Appareil (400) selon la revendication 9 ou 10, dans lequel le réservoir (402), les moyens de collecte (405), le couvercle (420) et/ou l'élément d'étanchéité (471) sont fabriqués à partir d'un matériau comprenant un ou plusieurs antioxydants qui agissent à l'encontre d'une détérioration oxydative d'un échantillon de sperme de mammifère.

12. Appareil (400) selon la revendication 11, dans lequel :

a. l'élément d'étanchéité (471) comprend une surface d'étanchéité (474) ;
b. le premier récipient (402) présente une surface intérieure (408) ; et
c. la surface d'étanchéité (474) de l'élément d'étanchéité (471) vient en prise avec la surface intérieure (408) du premier récipient (402) par positionnement, frottement, pression, aspiration, ou une combinaison de ceux-ci.

13. Appareil (400) selon la revendication 12, dans lequel la mise en prise de la surface d'étanchéité (474) de l'élément d'étanchéité (471) avec la surface intérieure (408) du premier récipient (402) forme une connexion étanche à l'air.

14. Appareil (400) selon la revendication 13, dans lequel les moyens d'étanchéité de réservoir (470) comprennent en outre un élément de positionnement (473).

15. Appareil (400) selon la revendication 14, dans lequel les moyens d'étanchéité de réservoir (470) comprennent en outre un élément de stabilisation (476).

16. Appareil (400) selon l'une quelconque des revendications 9 à 15, comprenant en outre une couche d'isolation, un espace d'air ou une combinaison de ceux-ci entre le premier récipient (402) et le second récipient (450).

17. Appareil (400) selon l'une quelconque des revendications 9 à 16, comprenant en outre une couche d'isolation entourant l'appareil.

FIG. 1B

FIG. 1A

100

176

173

174

172

171

170

105

110

102

**FIG. 2B**

100

171

173

172

176

174

102

**FIG. 2A**

FIG. 3B

FIG. 3A

FIG. 4A

FIG. 4B

EP 4 362 812 B1

FIG. 5B

FIG. 5A

FIG. 6B

FIG. 6A

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3744308 A1

- US 6864046 B